# EUROPEAN PATENT SPECIFICATION

(11) **EP 0 446 582 B1**
(45) Date of publication and mention of the grant of the patent: **04.01.1995**
(21) Application number: 91100777.1
(22) Date of filing: 22.01.1991
(51) Int. Cl.: C12N 15/23, C12P 21/02

(54) **Method for producing of recombinant human cysteineless gamma-interferon free of methionine at n-terminal**
Verfahren zur Herstellung von rekombinantem menschlichem gamma-Interferon ohne Cystein und ohne Methionin am N-Terminal
Méthode de production de l'interféron gamma humain recombinant sans cystéine et sa méthionine à l'extrémité NH2-terminale

(30) Priority: 24.01.1990 BG 91006/90
(43) Date of publication of application: 18.09.1991
(73) Proprietor: PHARMAGEN LTD., 1156 Sofia (BG)
(72) Inventor: Marekov, Ljuben Nikolaev, Sofia (BG); Vassileva, Rossiza Atanassova, Sofia (BG); Ivanov, Vesselin Penkov, Sofia (BG); Sarafova, Adriana Avram, Sofia (BG); Zanev, Rumen Georgiev, Sofia (BG); Ivanov, Ivan Georgiev, Sofia (BG); Ivanova, Vessela Stefanova, Sofia (BG)
(74) Representative: Bühling, Gerhard, Dipl.-Chem.

## Description

The invention refers to a method for producing of recombinant human cysteineless gamma-interferon free of methionine at N-terminal which is used in the medicine practice.

Methods are known for producing of recombinant human cysteine containing gamma-interferon(1, 2, 3) and cysteineless gamma-interferon (3, 4, 5) by expression in bacterial cells. In these methods the recombinant Interferon contains additionally methionine at its N-terminal which has remained as an initiating aminoacid in the bacterial synthesis.

There is known also a method for preparing of cysteineless gamma-interferon free of methionine at N-terminal (6) which is based on secretable expression in bacterial cells and on the use of alpha-amilase promoter and signal sequence for secretion.

The disadvantages of the known methods for producing of recombinant human cysteine-containing or cysteineless gamma-interferon (1 to 5) consist in that they lead to the obtaining of a protein containing an additional methionine at N-terminal the presence of which is unwanted since that makes the product immunogenic and its prolonged administration as therapeutic preparation might lead to the forming of specific antibodies.

A shortcoming of the method for producing of recombinant cysteineless gamma-interferon free of methionine at N-terminal (6) is that the removal of methionine is effected by secretion in the periplasmic space of the bacterium. During the transport there exist a danger of conformational modifications which might lead to immunogenity of the preparation. An other drawback is the exclusively low yield of interferon - 3000 000 U/l of culture.

With respect to the way of purification the known methods are based on immunosorption of gamma-interferon (7) or on the use of other chromatographic sorbents (2).

A disadvantage of immunosorption as method for purifying of gamma-interferon is the use of expensive, highly specific monoclonal antibodies the preparation of which requires a separate technology and there is a danger of contaminating the final product with transforming factors and viruses originating from hybridomic lines As a result can proceed conformation modifications of gamma-interferon(denaturation) which conduct to a decrease in biological activity.

A shortcoming of the chromatographic method without using of monoclonal antibodies (2) is that the method is based on chromatography on thiol-sepharose and it is applicable only for gamma-interferon containing cysteine.

Gamma-interferon as many other recombinant proteins can be easily aggregated in solutions which do not contain denaturating or chaotropic agents. After removal of guanidine chloride according to some of the well known methods (ultrafiltration, gel filtration, dialysis) gamma-interferon is aggregating. As a result the yield of soluble biologically active gamma-interferon can vary as a function of temperature, initial concentration of the protein, rate of exchange of the solvent, the presence of mono- and bivalent ions and probably to other for the time being unknown factors.

All this is hampering the standardizing of the technology, it decreases the yield and makes the final product more expensive.

The invention has as object to provide for a method for producing of recombinant human cysteineless gamma-interferon free of methionine at N-terminal which could warrant a high yield and a high degree of purity of the product as well as a complete dissolving in aqueous biologically compatible solutions containing no denaturating and chaotropic substances.

This object is attained by a method for producing of recombinant human cysteineless gamma-interferon free of methionine at N-terminal by means of expression of synthetic gene of gamma-interferon in microorganisms of genus E. coli in particular a strain E. coli obtained and recorded in the National Bank for deposition of industrial microorganisms and cell cultures under Nr 845 whereby the recombinant strain containing expression plasmide pIBM is cultivated in nutrition medium comprising tripitically ground pepton and yeast extract as sources of nitrogen being enriched with L-triptophan and L-methionine and in the presence of tetracycline.

The parameters of fermentation are so selected that they warrant a rapid appearance of exponential growth that are controlled during the proceeding of the process by addition of glucose. The interruption of aeration at the end of the process is favouring the removal of methionine at N-terminal as well as the forming of inclusion Bodies containing a high percentage of interferon in the intracellular space of the bacterium. After extraction the recombinant human cysteineless gamma-interferon is submitted to additional chromatographic treatment on a column of carboxymethylsepharose in order to remove the solvent and renaturation.

After producing interferon in gaunidine chloride with view to adsorb and retain the gamma-interferon on the ion-exchanger it is necessary that the concentration of guanidine chloride is below 0.35 M however not below 0.1 M. At a lower concentration of guanidine chloride the interferon is aggregating very rapidly.

In order to achieve good results the extract containing gamma-interferon is diluted with ice cold buffer with pH 5.0 to 8.5 and molarity 0.005 to 0.I M until is reached a concentration of guanidine chloride from 0.7 to 1.0 M. Immediately before charging the column with carboxymethyl sepharose the solutions of gamma-interferon and ice cold nuffer are mixed so that the dilution will provide for a final concentration of guanidine chloride of 0.35 M to be passed to the column. The guanidine chloride is removed by prolonged washing of the column with the respective buffer. Gamma-interferon is eluted by a gradient of sodium chloride from 0.1 to 0.6 M in the respective buffer.

The product is diluted with apyrogenic water to a physiological concentration of sodium chloride and it is mixed with a 10%-solution of dextrane up to a final concentration of 2% which is used as a stabilizer(3).

The advantage of the method according to the invention is that it is produced gamma-interferon the methionine of which at N-terminal has been removed intracellularly and not by secretion, that the yield of gamma-interferon is very high and exceeds 1 000 000 000 U/l of culture, that the produced recombinant human cysteineless gamma-interferon has a purity over 99.6%, that it retains its biological activity after lyophilizing in presence of a suitable stabilizer and it is soluble in aqueous solutions containing no denaturating or chaotropic agents, the technology avoids the applying of monoclonal antibodies for purifying of the recombinant protein that could contaminate the product in using cheap chromatographic technical devices being applied widely in the pharmaceutic practice and it offers a possibility for automated large scale production using a ion-exchanger which can be regenerated and sterilized for multiple industrial utilization.

The invention is illustrated by the following exemplary embodiment: Strain producer E.Coli LE 392, carrier of recombinant plasmid pIBM is stored at -70°C in 15% glycerine culture. After control of purity and activity the seed material is cultivated during 7 hours at 37°C by shaking in nutritive medium which is identical to the fermentation medium in volume 10% of the operating volume of the fermentator. The fermentation is accomplished in a standard type of a bacteriological fermentator equipped with a stirring device and having a working volume of 4l in a nutritive medium with the following composition( for 1l):

| | |
|---|---|
| Pepton, triptically grinded | 25 g |
| Yeast extract | 5 g |
| Sodium chloride | 5 g |
| Na₂HPO₄ | 6 g |
| K₂HPO₄ | 3 g |
| (NH₄)₂SO₄ | 3 g |
| MgSO₄ - 1M | 2 ml |
| CaCl₂ - 100 mM | 100 ml |
| Glucose 20% | 50 ml |
| L-triptophan 20 mkg/ml | 5 ml |
| L-methionine 20 mkg/ml | 5 ml |
| Tetracycline 10 mkg/ml | 1.2 ml |

The fermentation begins at an initial pH of 7.1 to 7.2 and an initial volume of aeration 0.5 l/1 l.volume/min, temperature 37.2°C and 400 rev/min of the stirring device.

When the culture attains the exponential phase of growth the volume of aeration is increased up to 1 l/ 1 l.volume/min. At the sixth hour from the begin of the fermentation to the culture are added 40 ml of 20% glucose and after termination of the exponential growth the aeration is interrupted and the culture is stirred during one more hour with 400 rev/min.

The cultural liquor is cooled and the biomass is collected. The yield is approximately 20g cell mass/ liter of culture. The biomass is broken and the gamma-interferon is extracted from the protein particles and then partially purified by fractional precipitation(2).

The obtained solution of gamma-interferon is diluted under stirring with ice cold 0.05M sodium acetate at pH 6.0 to concentration of guanidine chloride 0.7M. The thus prepared solution is loaded on column with carboxymethylsepharose(Farmazia,2.6 x10 cm, equilibrated in 0.05M sodium acetate buffer with pH 6.0) whereby immediately prior to the adsorption by means of pump and mixer the solution of gamma-interferon is diluted with ice cold buffer to a concentration of guanidine chloride 0.35M. Loading is effected with a rate 1100 ml/h. The column is washed out with 3 to 4 volumes of buffer and it is included a 20 min gradient 2x60 ml 0.1 to 0.6M sodium chloride in 0.05M sodium acetate buffer of pH 6.0 at a rate of elution 2 ml/min. Gamma-interferon is eluted at concentration 0.40 to 0.44M sodium chloride. The yield of pure gamma-interferon is nearly 1 mg pure protein for 1 g of bacterial mass.

The determining of the purity of the obtained gamma-interferon is performed according to two independent ways:
By electrphoresis in presence of sodium dodecyl sulphate and following densiometering of the strips and by chromatography at high pressure on column Pro-RPC(Farmazia, Sweden, 5x20 mm). Rate of elution 0.6 ml/min. Gradient -20 min, 0.01% trifluoracetic acid-100% acetonitrile in 0.01% trifluoracetic acid and adsorption at 280 nm-the purity of the product is 99.9% in using of electrophoresis and over 99.9% in applying of analytical chromatography.

The determining of aminoacid at N-terminal of the obtained recombinant cysteineless gamma-intergeron is performed by fluorescence labelling of aminoacid at N-terminal with dancyl chloride. As aminoacid at N-terminal of the thus produced recombinant human cysteineless gamma-interferon is identified glutamine. No presence of methionine or of other aminoacids has been established.

The determining of ratio soluble/aggregated interferon is carried out in centrifuging the interferon solution at 25 000 rev/min. The sediment(unsoluble interferon) is retained. The interferon soluble in the supernatant is precipitated with 20% trichloracetic acid. After centrifuging during 30 min at 10 000 rev/min the sediment is dissolved in buffer for electrophoresis and it is analyzed for polyacrylamidic gel in presence of sodium dodecyl sulphate parallelly with the precipitate of unsoluble interferon. After colouring of the gel the amount of protein in the strips is determined by densiometry.

The densiometric determination shows that all the protein(100%) is in a soluble state.

Concentration of gamma-interferon in a solution is determined and independently in using of molar extinction coefficient ε₂₈₀= 11 700(5).

Determining of biological activity is performed on the base of the protecting action of interferon with respect to the cytopatic effect of the virus of vesicular stomatite on human cell line WISH. The analysis is carried out in titration plates at a decreasing concentration of interferon. As a unit of activity is taken the concentration of interferon at which is observed a 50% protection of the cells. A laboratory standard calibrated according to an international standard for interferon-α is applied.

In cases when is studied the activity of a lyophilized preparation the ampules are reconstituted with 1 ml of apyrogenic water at different periods of time (7 days to 3 months) after lyophilizing. The interferon preparations studied manifest a specific activity approximating 5x10⁷ U/mg.

The method for producing of recombinant human cysteineless gamma-interferon free of methione at N-terminal is used for preparing the precious therapeutical means gamma-interferon. It provides for a product yield exceeding 1.10 U/l culture whereby there is no need of applying monoclonal antibodies for purifying. The thus obtained gamma-interferon in which methionine at N-terminal has been discarded intracellularly has a purity of over 99.6 %, it is maintaining its biological activity after lyophilizing and it is soluble in aqueous solutions. The method is accomplished in expressing the plasmid pIBM in straim Escherichia Coli LE 392 being recorded in the National Depositing Centre for industrial microorganisms and cellular cultures under Nr 845 and then cultivating it in a fermentator at temperatures from 36° to 38°C under conditions of aeration, thereafter the thus obtained product is purified, renaturated and eluted while the accompanying solvents and admixtures are removed.

## Claims

1. Method for producing of recombinant human cysteineless gamma-interferon free of methionine at N-terminal with participation of microorganisms of genus Escherichia Coli, characterized in that the plasmid pIBM - carrier of synthetic gene of recombinant human cysteineless gamma-interferon is expressed in strain-host E. coli recorded in the National Bank for depositing of industial microorganisms and cell cultures under Nr 845 being cultivated in a fermentator at temperature 36° to 38° C under conditions af aeration and the product obtained is purified and renaturated on a solid phase support whereby the accompanying solvents and admixtures are removed.

2. Method according to claim 1, characterized in that during fermentation with the cultures entering the exponential phase the aeration is augmented while at attaining of stationary phase aeration is discontinued at least one hour before collecting the biomass.

3. Method according to claim 1, characterized in that the solid phase support adsorbing interferon is a cation-exchange resin.

4. Method according to claim 1 and 3, characterized in that the cation-exchange resin is carboxymethylsepharose.

5. Method according to claim 1, characterized in that the adsorption of gamma-interferon on solid phase support is performed in a solution of guanidine chloride with concentration between 0.10 and 0.35 M.

6. Method according to claim 1, characterized in that washing for removing of the solvents is effected with apyrogenic water buffered with biologically compatible buffer compounds in concentrations from 0.005 to 0.050 M.

7. Method according to claim 1, characterized in that the removal of traces of bacterial proteins and other admixtures is effected with a solution of biologically compatible salts with concentration 0.10-0.35 M.

8. Method according to claim 1, characterized in that the elution of interferon is carried out with biologically compatible salts with concentration 0.4 to 0.55 M.

## Patentansprüche

1. Verfahren zur Herstellung von rekombinantem menschlichen Cystein-losen Gamma-Interferon ohne Methionin am N-Terminus mit der Beteiligung von Mikroorganismen der Gattung Escherichia Coli, dadurch gekennzeichnet, daß das Plasmid pIBM - Träger des synthetischen Gens von rekombinantem menschlichen Cystein-losen Gamma-Interferon in dem Wirtsstamm E. Coli, welcher in der Nationalen Bank zur Hinterlegung von industriellen Mikroorganismen und Zellkulturen unter Nr. 845 registriert ist, unter Züchtung in einem Fermentator bei Temperaturen von 36 ° bis 38 °C unter Belüftungsbedingungen exprimiert wird, und das erhaltene Produkt auf einem Feststoff-Träger gereinigt und renaturiert wird, wodurch die begleitenden Lösungsmittel und Beimischungen entfernt werden.

2. Verfahren nach Anspruch 1, dadurch gekennzeichnet, daß während der Fermentierung die Belüftung vergrößert wird, wenn die Kulturen in die exponentielle Phase eintreten, während bei Erreichen der stationären Phase die Belüftung mindestens eine Stunde unterbrochen wird, bevor die Biomasse gesammelt wird.

3. Verfahren nach Anspruch 1, dadurch gekennzeichnet, daß der Interferon adsorbierende Feststoff-Träger ein Kationen-Austauscherharz ist.

4. Verfahren nach einem der Ansprüche 1 und 3, dadurch gekennzeichnet, daß das Kationen-Austauscherharz Carboxymethylsepharose ist.

5. Verfahren nach Anspruch 1, dadurch gekennzeichnet, daß die Adsorption von Gamma-Interferon auf dem Feststoff-Träger in einer Lösung aus Guanidinchlorid mit einer Konzentration zwischen 0,10 und 0,35 M durchgeführt wird.

6. Verfahren nach Anspruch 1, dadurch gekennzeichnet, daß Waschen zum Entfernen der Lösungsmittel mit apyrogenischem Wasser, das mit biologisch verträglichen Puffer-Verbindungen in Konzentrationen von 0,005 bis 0,050 M gepuffert ist, bewirkt wird.

7. Verfahren nach Anspruch 1, dadurch gekennzeichnet, daß die Entfernung von Spuren bakterieller Proteine und weiterer Beimischungen mit einer Lösung aus biologisch verträglichen Salzen mit der Konzentration 0,10 bis 0,35 M bewirkt wird.

8. Verfahren nach Anspruch 1, dadurch gekennzeichnet, daß die Elution von Interferon mit biologisch verträglichen Salzen mit der Konzentration 0,4 bis 0,55 M durchgeführt wird.

## Revendications

1. Procédé de production d'interféron gamma humain recombinant exempt de cystéine et dépourvu de méthionine à l'extrémité N-terminale au moyen de micro-organismes appartenant au genre *Escherichia coli*, caractérisé en ce que le plasmide pIBM- porteur du gène synthétique de l'interféron gamma humain recombinant exempt de cystéine- est exprimé dans une souche hôte de *E. coli* consignée à la National Bank pour le dépôt de micro-organismes et de cultures cellulaires industriels sous le numéro 845 en mettant cette souche en culture dans une cuve de fermentation sous aération à une température de 36° à 38°C et en ce que le produit obtenu est purifié et renaturé sur un support formant phase solide de manière à éliminer les solvants et les mélanges également présents.

2. Procédé selon la revendication 1, caractérisé en ce que l'aération est renforcée pendant la fermentation lors de l'entrée des cultures en phase exponentielle et en ce qu'elle est interrompue pendant au moins une heure avant la collecte de la biomasse lorsque la phase stationnaire est atteinte.

3. Procédé selon la revendication 1, caractérisé en ce que le support formant phase solide pour l'adsorption de l'interféron est une résine échangeuse de cations.

4. Procédé selon les revendications 1 et 3, caractérisé en ce que la résine échangeuse de cations est la carboxyméthylsépharose.

5. Procédé selon la revendication 1, caractérisé en ce que l'adsorption de l'interféron gamma sur le support formant phase solide est effectuée dans une solution de chlorure de guanidine à une concentration comprise entre 0,10 et 0,35 M.

6. Procédé selon la revendication 1, caractérisé en ce qu'un lavage destiné à éliminer les solvants est réalisé avec de l'eau exempte de substances pyrogènes tamponnée avec des composés tampons biologiquement compatibles à des concentrations de 0,005 à 0,050 M.

7. Procédé selon la revendication 1, caractérisé en ce qu'une élimination des traces de protéines bactériennes et d'autres mélanges est réalisée au moyen d'une solution de sels biologiquement compatibles à une concentration de 0,10 à 0,35 M.

8. Procédé selon la revendication 1, caractérisé en ce que l'élution de l'interféron est réalisée avec des sels biologiquement compatibles à une concentration de 0,4 à 0,55 M.
